(19) Europäisches Patentamt
European Patent Office
Office européen des brevets

(11) Publication number: 0 141 199 A2

# (12) EUROPEAN PATENT APPLICATION

(21) Application number: 84110983.8

(22) Date of filing: 28.04.81

(51) Int. Cl.[4]: C 07 D 239/42
C 07 D 239/46, C 07 D 239/52
C 07 D 491/04, C 07 D 251/22
C 07 D 251/16, C 07 D 251/46
//(C07D491/04, 307:00, 239:00)

(30) Priority: 29.04.80 US 144856
25.03.81 US 244172
29.04.80 US 144857
25.03.81 US 244173
01.05.80 US 145582
25.03.81 US 244171

(43) Date of publication of application: 15.05.85 Bulletin 85/20

(84) Designated Contracting States: AT BE CH DE FR GB IT LI LU NL SE

(60) Publication number of the earlier application in accordance with Art. 76 EPC: 0 039 239

(71) Applicant: E.I. DU PONT DE NEMOURS AND COMPANY
1007 Market Street
Wilmington Delaware 19898(US)

(72) Inventor: Levitt, George
3218 Romilly Road
Wilmington Delaware 19810(US)

(74) Representative: Hildyard, Edward Martin et al,
Frank B. Dehn & Co. European Patent Attorneys Imperial House 15-19 Kingsway
London WC2B 6UZ(GB)

## (54) N-substituted sulfamoyl chlorides and preparation thereof.

(57) Sulfamoyl chlorides useful as intermediates for the preparation of herbicidal N-heterocyclic -N'-pyrrolesulfonamides have the general formula

$ClSO_2NHCONR_{10}$
|
$R_9$

wherein
$R_9$ is H, $CH_3$ or $OCH_3$;
$R_{10}$ is

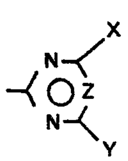 , 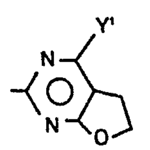 ,

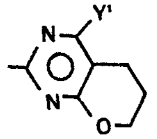 or 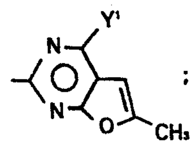 ;

X is $CH_3$ or $OCH_3$;
Y is H, $CH_3$, $OCH_3$, $OCH_2CH_3$, $OCH_2CF_3$, Cl, $CH_2OCH_3$, $CH_2OCH_2CH_3$ or $CF_3$;
Y' is H, $CH_3$, $OCH_3$, Cl or $OCH_2CH_3$; and
Z is CH, N, $CCH_3$, $CCH_2CH_3$, $CCH_2CH_2Cl$, CCl, CBr or CF.

The novel compounds may be prepared by reacting a corresponding heterocyclic amine of formula

$R_9R_{10}NH$

with chlorosulfonyl isocyanate.

EP 0 141 199 A2

4N136-913

"N-Substituted sulfamoyl chlorides and preparation thereof"

This invention relates to sulfamoyl chlorides having an N-(heterocyclic aminocarbonyl) substituent. The invention further provides a process for the preparation of these compounds.

The novel compounds of our invention have the general formula:

$$ClSO_2NHCON(R_9)R_{10}$$

(I)

wherein

$R_9$ is H, $CH_3$ or $OCH_3$;

$R_{10}$ is

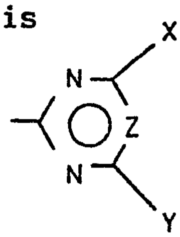

,

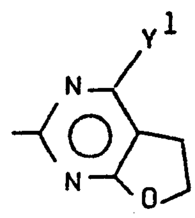

,

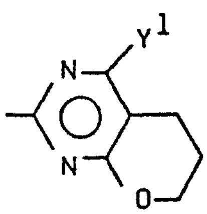

or

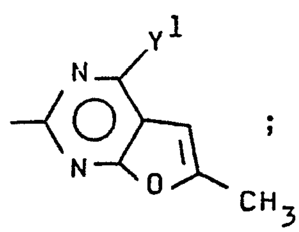

;

X is $CH_3$ or $OCH_3$;

Y is H, $CH_3$, $OCH_3$, $OCH_2CH_3$, $OCH_2CF_3$, Cl, $CH_2OCH_3$, $CH_2OCH_2CH_3$ or $CF_3$;

$Y^1$ is H, $CH_3$, $OCH_3$, Cl or $OCH_2CH_3$; and

Z is CH, N, $CCH_3$, $CCH_2CH_3$, $CCH_2CH_2Cl$, CCl, CBr or CF.

The compounds of general formula (I) can be reacted with pyrrole or substituted pyrroles under acylating conditions to prepare the N-heterocyclic-N'-pyrrolesulfonylureas disclosed and claimed in our EP-A-0,039,239. These sulfonylureas have potent herbicidal and plant growth regulant properties. The reader is referred to the said EP-A-0,039,239 for a full discussion of the utility of the sulfamoyl chlorides as intermediates for the preparation of herbicidal sulfonylureas. The high activity of the sulfonylureas is believed to be due, in large part, to the structure of the N-heterocyclic-aminocarbonylsulfamoyl moiety derived from the compounds of the present invention.

Preferred compounds of general formula (I) by reason of the superior activity of the sulfonylureas derived therefrom are:

(1) Compounds of the generic scope wherein $R_9$ is H or $CH_3$;

(2) Compounds of the <u>Preferred (1)</u> wherein Y is $CH_3$, $OCH_3$ or $OCH_2CH_3$; and Z is CH or N;

(3) Compounds of <u>Preferred (2)</u> wherein $Y^1$ is H, $CH_3$ or $OCH_3$;

(4) Compounds of <u>Preferred (1)-(3)</u> wherein $R_9$ is H; and

(5) Compounds of <u>Preferred</u> (4) wherein $R_{10}$ is

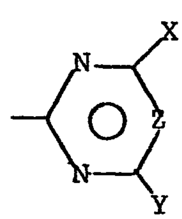

Specifically preferred for reasons of greatest activity of the derived sulfonylurea herbicides are:

[(4,6-dimethylpyrimidin-2-yl)aminocarbonyl]sulfamoyl chloride;

[(4-methoxy-6-methylpyrimidin-2-yl)aminocarbonyl]sulfamoyl

chloride;
[(4,6-dimethoxypyrimidin-2-yl)aminocarbonyl]sulfamoyl chloride;
[(4,6-dimethyl-1,3,5-triazin-2-yl)aminocarbonyl] sulfamoyl chloride;
[(4-methoxy-6-methyl-1,3,5-triazin-2-yl)aminocarbonyl]-sulfamoyl chloride;
[(4,6-dimethoxy-1,3,5-triazin-2-yl)aminocarbonyl]sulfamoyl chloride;
[(4-ethoxy-6-methyl-1,3,5-triazin-2-yl)aminocarbonyl]-sulfamoyl chloride;
[(5,6-dihydro-4-methylfuro[2,3-d]pyrimidin-2-yl) aminocarbonyl]sulfamoyl chloride;
[(5,6-dihydrofuro[2,3-d]pyrimidin-2-yl)aminocarbonyl]-sulfamoyl chloride;
[(5,6-dihydrofuro-4-methoxy[2,3-d]pyrimidin-2-yl)aminocarbonyl]sulfamoyl chloride;
[(4,6-dimethylfuro[2,3-d]pyrimidin-2-yl)aminocarbonyl]-sulfamoyl chloride;
[(4,6-dimethylpyrimidin-2-yl)(methyl)aminocarbonyl]-sulfamoyl chloride;
[(4,6-dimethoxypyrimidin-2-yl)(methyl)aminocarbonyl]-sulfamoyl chloride;
[(4-methoxy-6-methylpyrimidin-2-yl)(methyl)aminocarbonyl]-sulfamoyl chloride;
[(4,6-dimethyl-1,3,5-triazin-2-yl)(methyl)aminocarbonyl]-sulfamoyl chloride;
[(4,6-dimethoxy-1,3,5-triazin-2-yl)(methyl)aminocarbonyl]-sulfamoyl chloride; and
[(4-methoxy-6-methyl-1,3,5-triazin-2-yl)(methyl)aminocarbonyl]sulfamoyl chloride.

A general method for the preparation of the compounds of this invention is illustrated by the following equation:

$$R_9R_{10}NH \quad + \quad ClSO_2NCO \longrightarrow ClSO_2NHC(O)N(R_9)R_{10}$$

(I)

Two preferred reaction conditions for this synthesis will be designated Method A and Method B. In Method A, the reaction may be carried out at temperatures ranging from -80° to -40°C.

In Method B, the reaction is carried out at temperatures ranging from -10° to 10°C.

Synthesis - Method A

The heterocyclic amine $R_9R_{10}NH$ is suspended or dissolved in an inert organic solvent, such as dichloromethane, nitroethane, nitropropanes or tetrahydrofuran. Tetrahydrofuran or nitroethane are preferred. The reaction mixture is preferably maintained in an inert atmosphere at a temperature of -80° to -40°. One equivalent of chlorosulfonyl isocyanate, either neat or, if tetrahydrofuran is not the solvent, dissolved in the corresponding solvent, is contacted with the starting material at such a rate as to maintain the reaction temperature within the preferred range. The reaction proceeds rapidly and is allowed to continue for a period of 0.1 to 1 hour to ensure complete formation of product (I).

Synthesis - Method B

Method B is procedurally similar to Method A. For this method, suitable solvents include but are not limited to nitromethane, nitroethane, nitropropanes and dichloromethane; nitromethane is the solvent of choice. The reaction mixture is initially maintained at a temperature of -20° to 0° and the reaction is preferably carried out between -10° and 10°C. Again, a reaction time of 0.1 to 1 hour is allowed for the formation of product (I).

The synthesis of heterocyclic amine derivatives has been reviewed in "The Chemistry of Heterocyclic Compounds," a series published by Interscience Publ., New York and London. 2-Aminopyrimidines are described by D.J. Brown in "The Pyrimidines", Vol. XVI of the

above series. The 2-amino-1,3,5-triazines are reviewed by E.M. Smolin and L. Rapaport in "s-Triazines and Derivatives", Vol. XIII of the same series. The synthesis of triazines is also described by F.C. Schaefer, U.S.-A-3,154,547 and by K.R. Huffman and F.C. Schaefer, J. Org. Chem., 28 1812-1821 (1963). The synthesis of the bicyclic heterocyclic amines IV and V wherein $Y^1$ is as previously defined is described in our EP-A-0,015,683, published September 17, 1980.

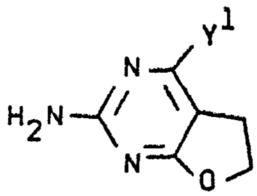

or

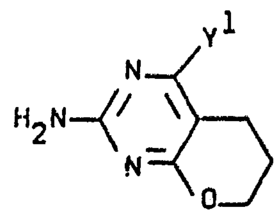

;

IV V

The pyrimidine intermediates of structure VI in which $Y^1$ is methyl, have been reported in the literature by E. Bisagni, et. al, Bull. Soc. Chim. Fr., 803 (1969). A more efficient procedure is depicted in the equation below.

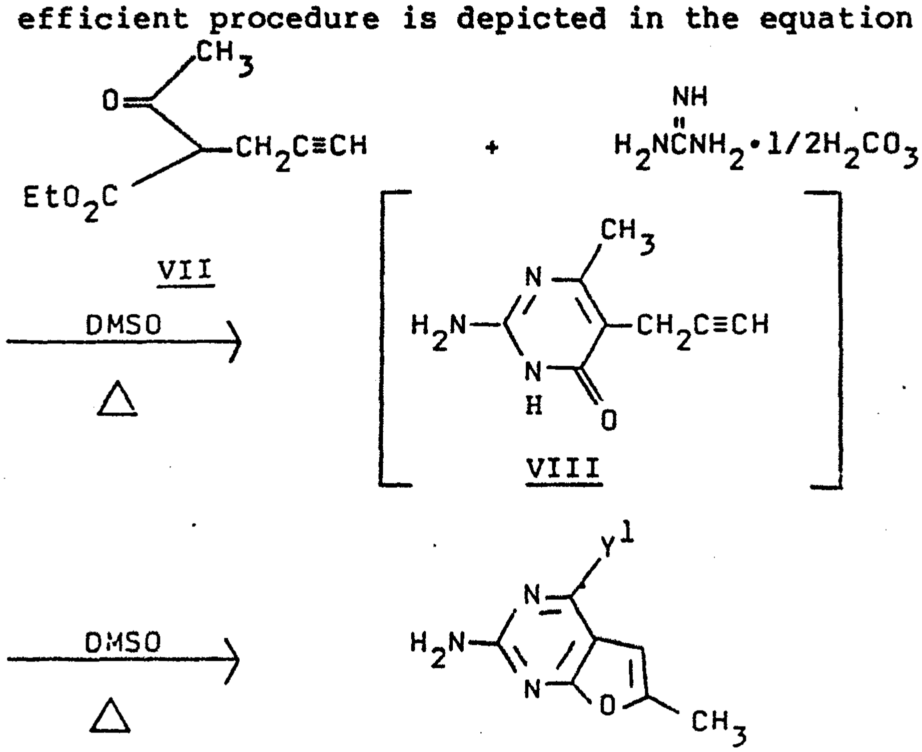


VII

VIII

VI

The known keto-ester precursor VII (e.g. J.F. Tinker and T.E. Whatmough, J. Amer. Chem. Soc., 74, 5235 (1952) is treated with excess guanidine carbonate

in an organic solvent, preferably a polar aprotic solvent such as dimethylsulfoxide (DMSO), dimethylformamide (DMF) or N,N-dimethylacetamide, at a temperature of 80° to 200°, preferably 100° to 160°, ambient pressure and preferably under an inert atmosphere to yield both VIII and VI (where $Y^1$ is $CH_3$). The products are isolated upon dilution of the reaction mixture with, for example, acetone and water successively. Higher reaction temperatures and longer reaction times (e.g., in DMSO at 130° to 150° for 2 to 8 hours) favour the production of VI over VIII.

The pyrimidine intermediates VI in which $Y^1$ is Cl may be prepared by condensing the known ethyl 2-carboethoxy-4-pentynoate IX with guanidine carbonate in an alcohol solvent such as ethanol to give the intermediate pyrimidine X as shown below.

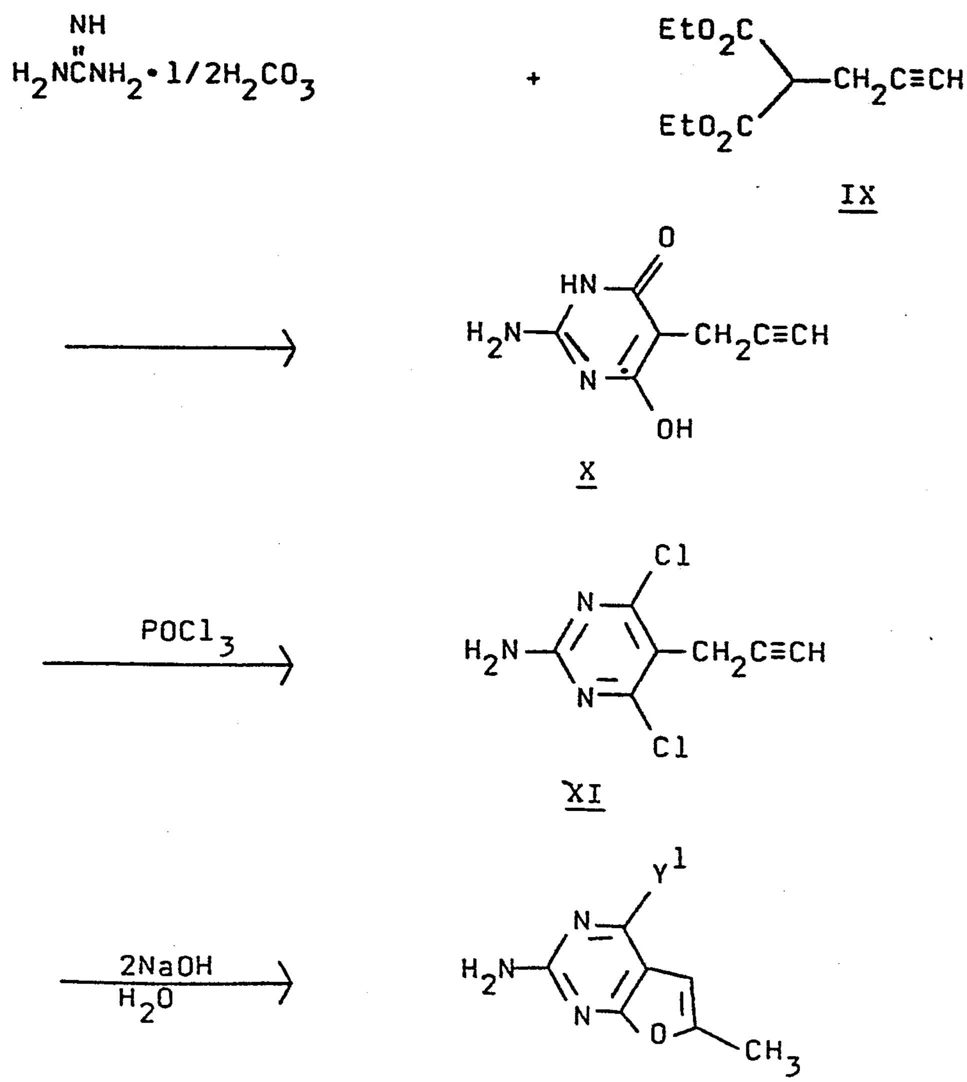


IX

X

XI

VI

Conversion to the dichloropyrimidine XI may be accomplished by heating X in phosphorous oxychloride. The product may be isolated by removal of the phosphorous oxychloride under reduced pressure, trituration of the residue with ice-water and filtration of the solid product. Contacting the dichloropyrimidine with two equivalents of an aqueous alkali metal hydroxide, such as sodium hydroxide, yields the furopyrimidine VI in which $Y^1$ is Cl. The reaction is best carried out in the presence of a solubilizing agent which is water miscible, such as *t*-butanol, dioxane or tetrahydrofuran, and at temperatures of 20 to 100° or at the boiling point of the solvent mixture used. The product may be isolated by cooling the mixture or further dilution with water to effect precipitation.

Compounds of Formula VI in which $Y^1$ is $OCH_3$ or $OCH_2CH_3$ may be prepared by treatment of the corresponding compound in which $Y^1$ is Cl with sodium methoxide in boiling methanol or with sodium ethoxide in refluxing ethanol, respectively. The product is obtained on evaporation of the alcohol solution, trituration of the residue with cold water and subsequent filtration.

Compounds of Formula VI in which $Y^1$ is H may be prepared by reaction of the corresponding compound in which $Y^1$ is Cl with a reducing agent such as zinc dust in acetic acid or *p*-toluenesulfonylhydrazide, the latter by a procedure similar to that described by Albert and Royer, J. Chem. Soc., 1148 (1949).

The aminoheterocyclic intermediates of $R_9R_{10}R_{11}$ in which $R_9$ is $CH_3$ may be prepared by the following procedure, or by obvious modifications:

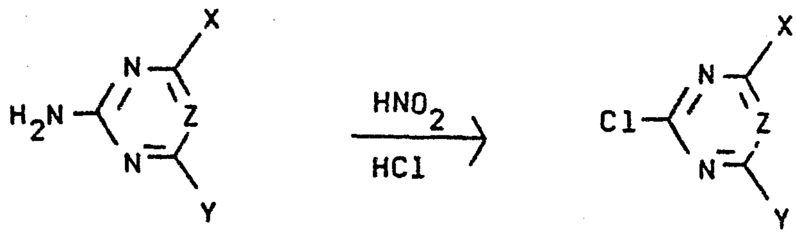


XII XIII

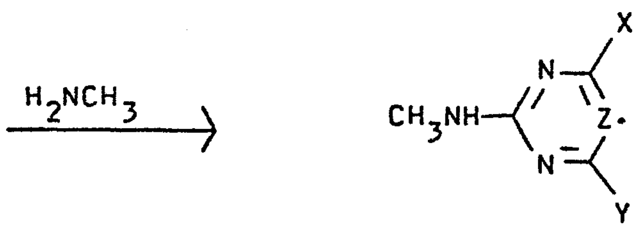


XIV

A solution of the amine XII in concentrated hydrochloric acid is treated with an aqueous sodium nitrite solution and the chloro compound XIII is isolated in the usual manner by filtration of the acidic solution (see for example, Bee and Rose, J. Chem. Soc. C., 2051 (1966) for the case in which Z is CH and X and Y are $OCH_3$). Displacement of the chlorine may be accomplished by heating with an excess of methylamine in water to obtain the methylamino heterocycle XIV.

N-Methoxyamino heterocycles can be prepared by procedures reported in the literature [see, for example, Belgian Patent 618,563 and J.T. Shaw, et al., J. Org. Chem., 27, 4054 (1962)] and illustrated below:

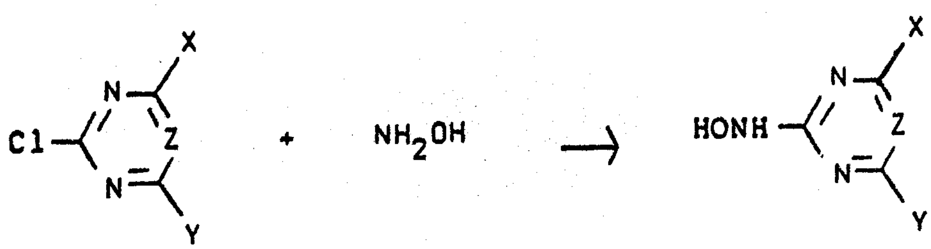


XIII

XV

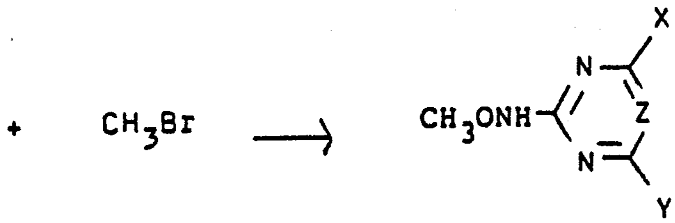


XVI

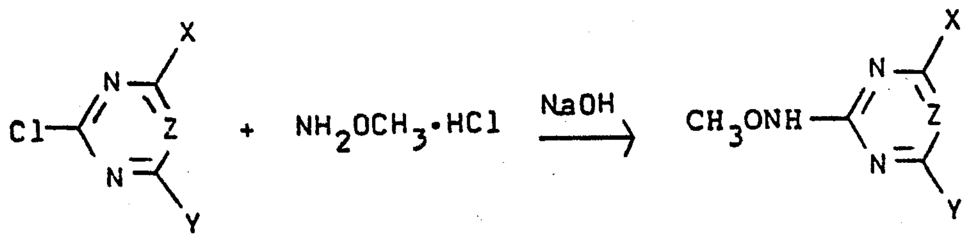


XIII

XVI

Chloro compound XIII is reacted with hydroxylamine to form derivative XV which may be alkylated with methyl bromide to afford the N-methoxy heterocyclic amine XVI. This compound may alternatively be prepared in one step by treatment of XIII and O-methylhydroxylamine hydrochloride with an alkali metal hydroxide such as sodium hydroxide.

The compounds of the invention and their preparation are further illustrated by the following Examples. In the following Examples, unless otherwise indicated, all parts are by weight and all temperatures in °C.

Example 1

Preparation of [(4,6-dimethylpyrimidin-2-yl)aminocarbonyl] sulfamoyl chloride.

To a mechanically stirred suspension of 12.3g (0.1 mole) of 2-amino-4,6-dimethylpyrimidine in 200 ml of dry dichloromethane maintained at 0° to 5°C under a nitrogen atmosphere was added dropwise over 30 minutes a solution of 9.2 ml (0.1 mole) of chlorosulfonyl isocyanate in 50 ml of ice-cold dichloromethane. After 30 minutes at 0° to 5°C the resulting yellow solution contained the title compound.

Example 2

Preparation of [(4,6-dimethylpyrimidin-2-yl)aminocarbonyl] sulfamoyl chloride.

Method A:

To a mechanically stirred mixture of 12.3 g (0.1 mole) of 2-amino-4,6-dimethylpyrimidine in 200 ml dry tetrahydrofuran at 0° to 5°C under a nitrogen atmosphere was added dropwise via syringe 9.2 ml (0.1 mole) of chlorosulfonyl isocyanate at such a rate to maintain the temperature below 5°C. After 10 minutes at 0° the solution contained the title compound.

Method B:

To a stirred mixture of 7.1 g (.057 mole) of 2-amino-4,6-dimethylpyrimidine in 35 ml of dry tetrahydrofuran at -78°C under a nitrogen atmosphere was added dropwise via syringe 5.0 ml (0.057 mole) of chlorosulfonyl isocyanate at such a rate to maintain the temperature below -50°C. The reaction was deemed to be complete after 30 minutes at -70°C.

Example 3

Preparation of [(4,6-dimethylpyrimidin-2-yl)aminocarbonyl]-sulfamoyl chloride.

To a stirred suspension of 2.58 g (0.021 mol) of 2-amino-4,6-dimethylpyrimidine in 75 ml dry nitromethane at -10° was added dropwise, via syringe, under nitrogen, 2.0 ml (0.023 mol) of chlorosulfonyl isocyanate at such a rate to maintain the temperature below 0°. The resulting clear solution was stirred 0.5 hour at -5 to -10° to form the title compound.

By using methods described generally above and illustrated in Examples 1-3, compounds as shown in the following tables can similarly be prepared. These tables are not meant to be all inclusive but only illustrative of the breadth of the invention.

Although the products in the following tables are not characterised by physical data, their identity can readily be verified by spectral methods or, in many cases, by reacting them with a pyrrole derivative to obtain one of the sulfonylureas prepared and characterised in our EP-A-0,039,239.

Table I

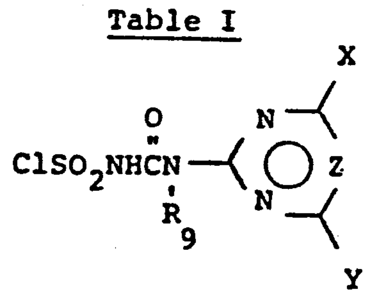

| $R_9$ | X | Y | Z |
|---|---|---|---|
| H | $CH_3$ | $OCH_3$ | CH |
| H | $OCH_3$ | $OCH_3$ | CH |
| H | $CH_3$ | $OC_2H_5$ | CH |
| H | $CH_3$ | Cl | CH |
| H | $CH_3$ | H | CH |
| H | $CH_3$ | $OCH_2CF_3$ | CH |
| H | $CH_3$ | $CH_2OCH_3$ | CH |
| H | $OCH_3$ | $CH_2OCH_3$ | CH |
| H | $OCH_3$ | $CH_2OC_2H_5$ | CH |
| H | $CH_3$ | $CF_3$ | CH |
| H | $OCH_3$ | $CF_3$ | CH |
| H | $CH_3$ | $CH_2OC_2H_5$ | CH |
| $CH_3$ | $OCH_3$ | $CH_3$ | CH |
| $CH_3$ | $OCH_3$ | $OCH_3$ | CH |
| $CH_3$ | $OCH_3$ | $OC_2H_5$ | CH |
| H | $OCH_3$ | Cl | CH |
| H | $OCH_3$ | $CH_2OCH_3$ | CH |
| H | $OCH_3$ | $OCH_2CF_3$ | CH |
| H | $CH_3$ | $CH_3$ | CCl |
| H | $CH_3$ | $OCH_3$ | CCl |
| H | $OCH_3$ | $OCH_3$ | CCl |
| H | $CH_3$ | $CH_3$ | $CCH_3$ |
| H | $CH_3$ | H | $CCH_3$ |
| H | $OCH_3$ | H | $CCH_3$ |
| H | $CH_3$ | H | CCl |

Table I (continued)

| $R_9$ | X | Y | Z |
|---|---|---|---|
| H | $CH_3$ | $CH_3$ | CF |
| H | $CH_3$ | $CH_3O$ | CF |
| H | $CH_3$ | $CH_3$ | CBr |
| H | $CH_3$ | H | CBr |
| H | $CH_3$ | H | $CCH_2CH_3$ |
| H | $CH_3$ | H | $CCH_2CH_2Cl$ |
| H | $CH_3$ | $CH_3$ | $CCH_2CH_2Cl$ |
| H | $CH_3$ | H | CF |
| H | $OCH_3$ | H | CH |
| H | $OCH_3$ | Cl | CH |
| $CH_3$ | $CH_3$ | $CH_3$ | CH |
| $OCH_3$ | $CH_3$ | $CH_3$ | CH |
| $OCH_3$ | $CH_3$ | $OCH_3$ | CH |
| $OCH_3$ | $OCH_3$ | $OCH_3$ | CH |

Table II

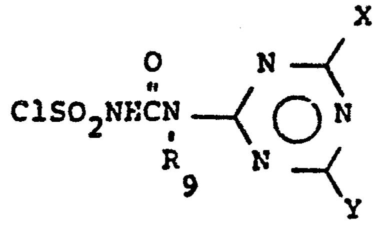

| $R_9$ | X | Y |
|---|---|---|
| H | $CH_3$ | $CH_3$ |
| H | $CH_3$ | $OCH_3$ |
| H | $OCH_3$ | $OCH_3$ |
| H | $CH_3$ | $OCH_2CH_3$ |
| H | $CH_3$ | Cl |
| H | $CH_3$ | H |
| H | $CH_3$ | $OCH_2CF_3$ |
| H | $CH_3$ | $CH_2OCH_3$ |
| H | $OCH_3$ | $CH_2OCH_3$ |
| H | $OCH_3$ | $CH_2OCH_2CH_3$ |
| H | $CH_3$ | $CF_3$ |
| H | $OCH_3$ | $CF_3$ |
| H | $CH_3$ | $CH_2OC_2H_5$ |
| $CH_3$ | $OCH_3$ | $CH_3$ |
| $CH_3$ | $OCH_3$ | $OCH_3$ |
| $CH_3$ | $OCH_3$ | $OC_2H_5$ |
| H | $OCH_3$ | Cl |
| H | $OCH_3$ | $OCH_2CF_3$ |
| $OCH_3$ | $CH_3$ | $CH_3$ |
| $OCH_3$ | $OCH_3$ | $CH_3$ |
| $OCH_3$ | $OCH_3$ | $OCH_3$ |
| $OCH_3$ | $CH_3$ | $OCH_2CH_3$ |
| $OCH_3$ | $CH_3$ | $OCH_2CF_3$ |
| $OCH_3$ | $CH_3$ | $CH_2OCH_3$ |
| $OCH_3$ | $CH_3$ | $OCH_2CF_3$ |
| $CH_3$ | $CH_3$ | $CH_3$ |

Table III

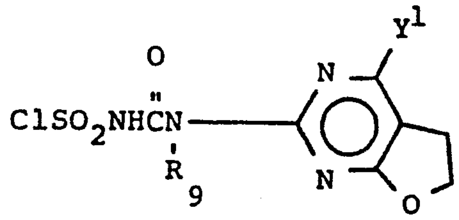

| $R_9$ | $Y^1$ |
|---|---|
| H | H |
| H | $OCH_3$ |
| H | $CH_3$ |
| H | Cl |
| H | $OCH_2CH_3$ |
| $OCH_3$ | $OCH_3$ |
| $OCH_3$ | $CH_3$ |
| $OCH_3$ | Cl |
| $OCH_3$ | $OC_2H_5$ |
| $OCH_3$ | H |
| $CH_3$ | $CH_3$ |
| $CH_3$ | $OCH_3$ |
| $CH_3$ | $OCH_2CH_3$ |
| $CH_3$ | Cl |
| $CH_3$ | H |

Table LV

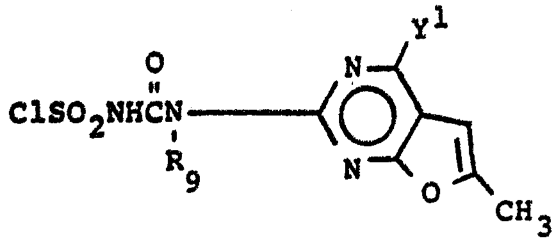

| $R_9$ | $Y^1$ |
|---|---|
| H | $CH_3$ |
| H | $OCH_3$ |
| $CH_3$ | $CH_3$ |
| $CH_3$ | $OCH_3$ |
| $OCH_3$ | $CH_3$ |
| $OCH_3$ | $OCH_3$ |
| H | H |
| $CH_3$ | H |
| $OCH_3$ | H |
| H | Cl |
| $CH_3$ | Cl |
| $OCH_3$ | Cl |
| H | $OCH_2CH_3$ |
| $CH_3$ | $OCH_2CH_3$ |
| $OCH_3$ | $OCH_2CH_3$ |

Table V

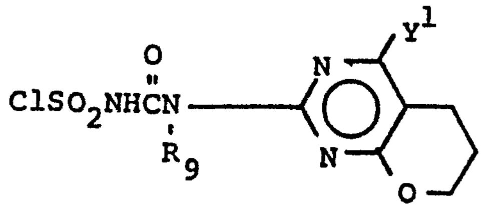

| $R_9$ | $Y^1$ |
|---|---|
| H | $CH_3$ |
| H | $OCH_3$ |
| $CH_3$ | $CH_3$ |
| $CH_3$ | $OCH_3$ |
| $OCH_3$ | $CH_3$ |
| $OCH_3$ | $OCH_3$ |
| H | H |
| $CH_3$ | H |
| $OCH_3$ | H |
| H | Cl |
| $CH_3$ | Cl |
| $OCH_3$ | Cl |
| H | $OCH_2CH_3$ |
| $CH_3$ | $OCH_2CH_3$ |
| $OCH_3$ | $OCH_2CH_3$ |

Claims:

1) A sulfamoyl chloride having the general formula:

$$ClSO_2NHCON(R_9)R_{10}$$

(I)

wherein

$R_9$ is H, $CH_3$ or $OCH_3$;

$R_{10}$ is

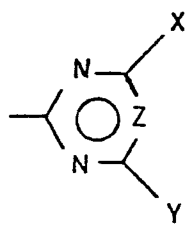
,
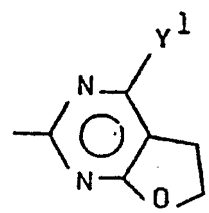
,

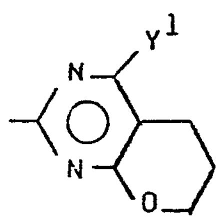
or
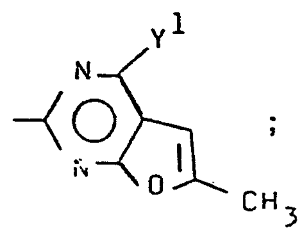
;

X is $CH_3$ or $OCH_3$;

Y is H, $CH_3$, $OCH_3$, $OCH_2CH_3$, $OCH_2CF_3$, Cl, $CH_2OCH_3$, $CH_2OCH_2CH_3$ or $CF_3$;

$Y^1$ is H, $CH_3$, $OCH_3$, Cl or $OCH_2CH_3$; and

Z is CH, N, $CCH_3$, $CCH_2CH_3$, $CCH_2CH_2Cl$, CCl, CBr or CF.

2) A compound of claim 1 wherein $R_9$ is H or $CH_3$.

3) A compound of claim 2 wherein Y is $CH_3$, $OCH_3$ or $OCH_2CH_3$; and Z is CH or N.

4) A compound of claim 3 wherein $Y^1$ is H, $CH_3$ or $OCH_3$.

5) A compound of any of claims 2-4 wherein $R_9$ is H.

6) A compound of claim 5 wherein $R_{10}$ is

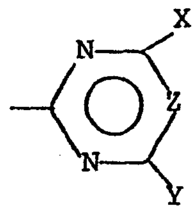

7) The compound of claim 1 which is [(4,6-dimethylpyrimidin-2-yl)aminocarbonyl]sulfamoyl chloride.

8) The compound of claim 1 which is [(4-methoxy-6-methylpyrimidin-2-yl)aminocarbonyl]sulfamoyl chloride.

9) The compound of claim 1 which is [(4,6-dimethoxypyrimidin-2-yl)aminocarbonyl]sulfamoyl chloride.

10) The compound of claim 1 which is [(4,6-dimethyl-1,3,5-triazin-2-yl)aminocarbonyl]sulfamoyl chloride.

11) The compound of claim 1 which is [(4-methoxy-6-methyl-1,3,5-triazin-2-yl)aminocarbonyl]sulfamoyl chloride.

12) The compound of claim 1 which is [(4,6-dimethoxy-1,3,5-triazin-2-yl)aminocarbonyl]sulfamoyl chloride.

13) The compound of claim 1 which is [(4-ethoxy-6-methyl-1,3,5-triazin-2-yl)aminocarbonyl]sulfamoyl chloride.

14) A compound of claim 1 which is selected from [(5,6-dihydro-4-methylfuro[2,3-d]pyrimidin-2-yl)aminocarbonyl]sulfamoyl chloride;
[(5,6-dihydrofuro[2,3-d]pyrimidin-2-yl)aminocarbonyl]-sulfamoyl chloride;
[(5,6-dihydrofuro-4-methoxy[2,3-d]pyrimidin-2-yl)aminocarbonyl]sulfamoyl chloride; or
[(4,6-dimethylfuro[2,3-d]pyrimidin-2-yl)aminocarbonyl]-sulfamoyl chloride.

15) A compound of claim 1 which is selected from [(4,6-dimethylpyrimidin-2-yl)(methyl)aminocarbonyl]-sulfamoyl chloride;

[(4,6-dimethoxypyrimidin-2-yl)(methyl)aminocarbonyl]sulfamoyl chloride;

[(4-methoxy-6-methylpyrimidin-2-yl)(methyl)aminocarbonyl]-sulfamoyl chloride;

[(4,6-dimethyl-1,3,5-triazin-2-yl)(methyl)aminocarbonyl]-sulfamoyl chloride;

[(4,6-dimethoxy-1,3,5-triazin-2-yl)(methyl)aminocarbonyl]-sulfamoyl chloride; or

[(4-methoxy-6-methyl-1,3,5-triazin-2-yl)(methyl)aminocarbonyl]sulfamoyl chloride.

16) A compound of claim 1 wherein

$R_9$ is H or $CH_3$; and

$R_{10}$ is

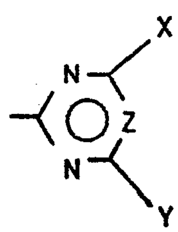

or

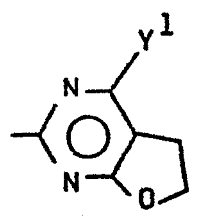

17) A method of making a compound of claim 1 which comprises reacting a corresponding heterocyclic amine of formula

$$R_9R_{10}NH$$

with chlorosulfonyl isocyanate.

18) A method according to claim 17 wherein the reaction is carried out in an inert solvent at a temperature in the range from -80°C to 10°C.

Claims:

1. A process for the preparation of a sulfamoyl chloride having the general formula:

$$ClSO_2NHCONR_{10}$$
$$R_9$$

(I)

wherein

$R_9$ is H, $CH_3$ or $OCH_3$;

$R_{10}$ is

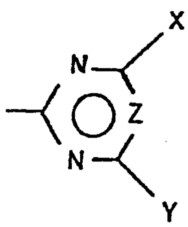

,

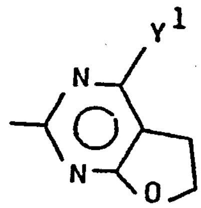

,

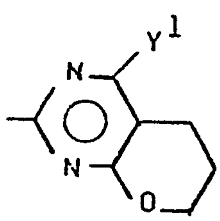

or

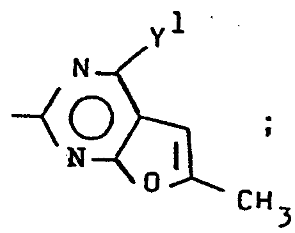

;

X is $CH_3$ or $OCH_3$;

Y is H, $CH_3$, $OCH_3$, $OCH_2CH_3$, $OCH_2CF_3$, Cl, $CH_2OCH_3$, $CH_2OCH_2CH_3$ or $CF_3$;

$Y^1$ is H, $CH_3$, $OCH_3$, Cl or $OCH_2CH_3$; and

Z is CH, N, $CCH_3$, $CCH_2CH_3$, $CCH_2CH_2Cl$, CCl, CBr or CF;

which comprises reacting a corresponding heterocyclic amine of formula

$$R_9R_{10}NH$$

with chlorosulfonyl isocyanate.

2) A method according to claim 1 wherein the reaction is carried out in an inert solvent at a temperature in the range from -80°C to 10°C.

3) A method according to claim 2 wherein said solvent is selected from dichloromethane, nitromethane, nitroethane, nitropropane or tetrahydrofuran.

4) A process of claim 1, 2 or 3 wherein

$R_9$ is H or $CH_3$; and

$R_{10}$ is

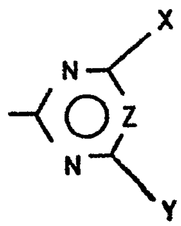

or

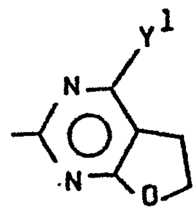

5) A process of any of claims 1 to 4 wherein $R_9$ is H or $CH_3$.

6) A process of claim 5 wherein Y is $CH_3$, $OCH_3$ or $OCH_2CH_3$; and Z is CH or N.

7) A process of claim 6 wherein $Y^1$ is H, $CH_3$ or $OCH_3$.

8) A process of any of claims 5-7 wherein $R_9$ is H.

9) A process of claim 8 wherein $R_{10}$ is

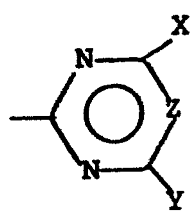

10) A process of claim 1 wherein the product is a compound selected from

[(4,6-dimethylpyrimidin-2-yl)aminocarbonyl]sulfamoyl chloride;

[(4-methoxy-6-methylpyrimidin-2-yl)aminocarbonyl]sulfamoyl chloride;

[(4,6-dimethoxypyrimidin-2-yl)aminocarbonyl]sulfamoyl chloride;

[(4,6-dimethyl-1,3,5-triazin-2-yl)aminocarbonyl]sulfamoyl chloride;
[(4-methoxy-6-methyl-1,3,5-triazin-2-yl)aminocarbonyl] sulfamoyl chloride;
[(4,6-dimethoxy-1,3,5-triazin-2-yl)aminocarbonyl]sulfamoyl chloride; or
[(4-ethoxy-6-methyl-1,3,5-triazin-2-yl)aminocarbonyl] sulfamoyl chloride.

11) A process of claim 1 wherein the product is a compound selected from
[(5,6-dihydro-4-methylfuro[2,3-d]pyrimidin-2-yl)aminocarbonyl]sulfamoyl chloride;
[(5,6-dihydrofuro[2,3-d]pyrimidin-2-yl)aminocarbonyl]-sulfamoyl chloride;
[(5,6-dihydrofuro-4-methoxy[2,3-d]pyrimidin-2-yl)aminocarbonyl]sulfamoyl chloride; or
[(4,6-dimethylfuro[2,3-d]pyrimidin-2-yl)aminocarbonyl]-sulfamoyl chloride;

12) A process of claim 1 wherein the product is a compound selected from
[(4,6-dimethylpyrimidin-2-yl)(methyl)aminocarbonyl]-sulfamoyl chloride;
[(4,6-dimethoxypyrimidin-2-yl)(methyl)aminocarbonyl]sulfamoyl chloride;
[(4-methoxy-6-methylpyrimidin-2-yl)(methyl)aminocarbonyl]-sulfamoyl chloride;
[(4,6-dimethyl-1,3,5-triazin-2-yl)(methyl)aminocarbonyl]-sulfamoyl chloride;
[(4,6-dimethoxy-1,3,5-triazin-2-yl)(methyl)aminocarbonyl]-sulfamoyl chloride; or
[(4-methoxy-6-methyl-1,3,5-triazin-2-yl)(methyl)amino-carbonyl]sulfamoyl chloride.